# EUROPEAN PATENT APPLICATION

(11) **EP 0 704 221 A2**
(43) Date of publication of application: **03.04.1996**
(21) Application number: 95306945.7
(22) Date of filing: 29.09.1995
(51) Int. Cl.: A61K 47/48

(54) **A conjugate useful in gene transfer and a method of producing the same**

(30) Priority: 29.09.1994 JP 270102/94
(71) Applicant: AJINOMOTO CO., INC., Tokyo 104 (JP)
(72) Inventor: Takahara, Yoshiyuki, Central Res., Ajinomoto Co., Kawasaki-shi, Kanagawa (JP); Yamada, Naoyuki, Central Res., Ajinomoto Co., Kawasaki-shi, Kanagawa (JP); Motoki, Masao, Central Res., Ajinomoto Co., Kawasaki-shi, Kanagawa (JP)
(74) Representative: Silverman, Warren

(57) **Abstract**

The present invention relates to a process for producing a molecule for binding a nucleic acid, which molecule comprises a biologically-active peptide or protein having at least one glutamine residue linked to a high molecular weight substance containing an amino acid donor and having the ability to bind a nucleic acid. The biologically-active peptide or protein and high molecular weight substance are reacted in the presence of a transglutaminase to form an acid amide linkage between the amino group of the amino acid donor and the γ-position of the glutamine residue.

## Description

The present invention relates to a conjugate useful in gene transfer and a method of producing the same. The conjugate comprises a biologically-active peptide or protein linked to a high molecular weight substance.

A large number of biologically-active peptides or proteins are known. These will hereinafter also be referred to as "biologically-active peptides". Examples of biologically-active peptides are naturally-occurring biologically-active peptides which include, for example, peptides which exhibit a physiological action in the human body, such as interleukin (IL)-1, - 2, -3, -4, -5, -6, -7, -8, -9, -10, -11 and -12, G-CSF, GM-CSF, M-CSF, erythropoietin, stem cell factor (SCF), mpl-ligand, α-, β- and γ-interferons, somatostatin, vasopressin, insulin, growth hormone and substance P; peptides obtained by modifying the above-mentioned peptides; substances derived from animals, such as bombesin; substances derived from microorganisms, such as diphtheria toxin; and substances derived from plants, such as lectin. Antibodies are also biologically-active peptides and include not only human monoclonal antibodies but also animal monoclonal antibodies. Many biologically-active peptides bind specifically to corresponding receptors in cells to activate their physiological functions. Antibodies bind specifically to their corresponding antigens.

The soluble receptor includes a receptor present in the human body and a receptor which is artificially produced by a gene recombination method. These soluble receptors, like the receptors on the membranes of the human body, bind specifically to ligands.

It has been considered that specific biologically-active peptides be used as targeting molecules utilizing the above-mentioned specific binding. For example, a method has been attempted in which diphtheria toxin is linked to an IL-6 molecule, and specifically sent to tumour cells having an IL-6 receptor, thereby to kill the tumour cells. Further, a method in which an anti-tumour agent is linked to a monoclonal antibody that recognizes a cancer-specific antigen thereby to send the anti-tumour agent specifically to the tumour cells has been also developed as an effective method.

Furthermore, a method using biologically-active peptides in a drug delivery system (DDS) is also important in the field of gene therapy. George Y. Wu et al. have developed a method of gene therapy in which polylysine (having a positive charge) is linked, by a chemical method, to a biologically-active peptide, such as asialoorosomucoid, to form a conjugate. This conjugate then binds a DNA plasmid including a gene (having a negative charge) to send the gene to a hepatocyte having a asialoglycoprotein receptor (J. Biol. Chem., 236, 14621, 1988). This conjugate can transfer the gene into the liver through intravenous injection, and is expected to be an efficient gene delivery system. Diseases to be treated by this gene therapy method depend on the delivered gene. In one method, a viral thymidine kinase gene is transferred into the liver, an anti-viral drug ganciclovir to be activated by the thymidine kinase is transferred, and the anti-viral drug is activated only in the liver, whereby the viral disease is treated with fewer side effects.

When a nucleic acid is administered to the human body for gene therapy, the nucleic acid is enzymatically decomposed rapidly or cannot enter a cell easily. Further, when a gene is inserted into the genome of a cell by the above-mentioned gene therapy, the cell is often impaired at some insertion portion, or the substance expressed by the transferred gene sometimes impairs the cell. Accordingly, the following features are desirable:
i) the nucleic acid is sent to the cell without the nucleic acid being decomposed;
ii) the nucleic acid is transferred into the cell at a high rate; and
iii) the nucleic acid is transferred as much as possible to the specific cell requiring therapy.

As one solution to the above-mentioned problems, the nucleic acid is bound by means of a polymer to suppress the decomposition of the nucleic acid. As another solution, the ligand to be bound to a specific receptor on the cell is linked to the nucleic acid/polymer complex, and the nucleic acid is transferred into the specific cell type by means of endocytosis through ligand-receptor binding. In this case, it is necessary to link the polymer binding the nucleic acid to the ligand or biologically-active peptide.

In general, when a biologically-active peptide is linked to the polymer by means of a chemical reaction, their biological activities tend to be impaired. In addition, by-products are formed through various side reactions, making it hard to maintain the quality of the drugs to be produced, which require a constant high level of quality. The development of a practical method in which a high molecular weight substance having the ability to bind a nucleic acid is linked specifically to a biologically-active peptide under mild conditions is in demand. Also, side reactions should be avoided in this process.

According to a first aspect of the present invention, there is provided a conjugate of (a) a biologically-active peptide or protein having at least one glutamine residue and (b) a high molecular weight substance containing an amino acid donor and having the ability to bind a nucleic acid, the biologically-active peptide or protein being linked to the high molecular weight substance by an acid-amide linkage between the amino group of the amino acid donor and the γ-position of the glutamine residue.

The high molecular weight substance may be polylysine and the biologically-active protein may be interleukin-6 or interleukin-2.

According to a second aspect of the present invention, there is provided a process for producing a conjugate in accordance with the first aspect of the present invention, comprising reacting said biologically-active peptide or protein with said high molecular weight substance in the presence of a transglutaminase.

According to a third aspect of the present invention, there is provided a complex comprising a conjugate in accordance with the first aspect of the present invention or a conjugate produced by the method of the second aspect of the present invention, and a nucleic acid, preferably DNA.

The present invention also provides a pharmaceutical composition for gene transfer therapy, which comprises a conjugate in accordance with the first aspect of the present invention or a conjugate produced by the method of the second aspect of the present invention, and a pharmaceutically-acceptable carrier.

The present also provides a pharmaceutical composition for gene transfer therapy, which comprises a complex in accordance with the third aspect of the present invention and a pharmaceutically-acceptable carrier.

In one embodiment, the biologically-active peptide or protein can bind to a specific receptor of a cell in the human body. Thus the conjugate can bind to the cell having the specific receptor and can transfer into the cell a nucleic acid bound to the conjugate. Gene therapy can therefore be carried out in which a gene is transferred into a specific type of cell, a specific organ or tumour cells of the human body.

The present invention relates to a process for producing a protein for the inclusion of a nucleic acid, which comprises reacting a biologically-active peptide or protein having at least one glutamine residue with a high-molecular substance containing an amino acid donor and having the ability to include the nucleic acid, in the presence of a transglutaminase to form an acid-amide linkage with the amino group of the amino acid donor in the acid amide at the γ-position of the glutamine residue.

For a better understanding of the invention, and to show how the same may be carried into effect, reference will now be made, by way of example only, to the accompanying drawings, in which:-
Fig. 1 shows a pattern of chromatography of a reaction solution of polylysine and IL-6;
Fig. 2 shows a pattern of chromatography of purified polylysine-bound IL-6;
Fig. 3 shows results of in-vitro activity of IL-6 and polylysine-bound IL-6; and
Fig. 4 shows an electrophoresis pattern for the formation of a complex of polylysine-bound IL-6 and DNA.

The present inventors have found that a high molecular weight substance having the ability to bind a nucleic acid can be linked to a biologically-active peptide under mild conditions using a transglutaminase.

Transglutaminase (EC 2.3.2.13) (family name: protein-glutamine: γ-glutamyltransferase) is a known enzyme by which a glutamine residue of a peptide or protein can be specifically linked to an amino group of a substance. The transglutaminase has been reported in a variety of documents, for example: (a) J.E. Folk et al., Adv. Protein Chem. 31, 1-133, 1977; (b) J.E. Folk et al., Adv. Enzymol, 38, 109-191, 1973; (c) H. Bohn et al., Mol. Cell Biochem. 20, 67-75, 1978; (d) L. Lorand et al., Handbook of Biochemistry and Molecular Biology, vol. 2, Proteins, ed G.D. Fasman, pp. 669-685, Cleveland: CRC. 3rd ed.; (e) Guinea pig and rabbit liver transglutaminase: T. Abe et al., Biochemistry, 16, 5495-5501 (1977); (f) Human red blood cells transglutaminase: S.C. Brenner et al., Biochem. Biophys. Acta., 522, 74-83, 1978; and (g) Rat coagulating gland transglutaminase: J. Wilson et al., Fed. Proc., 38, 1809 (Abstr.), (1979).

Various kinds of transglutaminases have been known according to their origin. Examples thereof include a transglutaminase derived from micro-organisms (bacterial transglutaminase, hereinafter simply referred to as "BTG", which is reported by H. Ando et al., Agric. Biol. Chem., 53(10), 2613-2617 (1989), and K. Washizu et al., Biosci. Biotech. Biochem., 58(1), 82-87 (1994)), and mammalian transglutaminases, such as liver transglutaminase, plasma factor XIIIa, platelet and placental factor XIIIa, hair-follicle transglutaminase, epidermal transglutaminase and prostate transglutaminase. Any of these transglutaminases can be used in the present invention. However, BTG is preferable.

In the reaction catalysed by the transglutaminase, the linking is conducted only in a specific position. Accordingly, few kinds of by-products are formed, and therefore the reaction can be easily controlled. Further, because of the enzymatic reaction, the following advantages are obtained by comparison to a chemical reaction:
i) there is a strong possibility that a conjugate is formed at a special position without a reduction in the function of the substance to be linked;
ii) since the reaction is conducted under physiological conditions, the substance to be linked is not denatured; and
iii) as the level of quality is maintained, a high-quality drug can be produced; the products can constantly have a unique, defined structure.

Meanwhile, it is also known to link proteins to each other using a recombinant DNA method in which genes are combined with each other. Compared to the recombinant DNA method, the process using the transglutaminase possesses the following advantages:
i) substances which cannot be directly synthesized from DNA, such as a sugar chain, glycoprotein, glycolipid, PEG (polyethylene glycol) and the like can also be linked;
ii) the recombinant DNA method merely produces a fusion protein in which a protein is bound to the N-terminal or C-terminal of another protein; and
iii) since the recombinant DNA method merely produces a fusion protein in which a protein is bound to the N-terminal or C-terminal of another protein, the active centre is present in the terminal of the protein. Thus, there is a strong possibility that the fusion protein loses its activity.

On the other hand, when using a transglutaminase, the substance having the amino group is linked to the amino group of glutamine only. Thus, a conjugate different from that obtained by the recombinant DNA method is obtained, wherein the activities of both of the components are maintained upon controlling the linking by BTG.

The high molecular weight compound having the ability to bind the nucleic acid can be linked to the biologically-active peptide under the enzymatic reaction conditions of the transglutaminase used.

For example, in the case of using BTG, the reaction can be carried out at a temperature in the range of 4 to 55 °C, preferably of 30 to 50 °C and at a pH in the range of 5 to 8, preferably of 6 to 7.

The transglutaminase activity referred to in the present invention can be measured as follows. A reaction system containing benzyloxycarbonyl-L-glutamineglycine and hydroxylamine as substrates is reacted with animal transglutaminase in a tris-buffer (pH 6.0) at 37 °C in the presence of 5 mM Ca⁺. In the case of with microorganism transglutaminase, the reaction is carried out in the absence of 5 mM Ca⁺. The thus obtained hydroxamic acid is formed into an iron complex in the presence of trifluoroacetic acid. Then, the absorbance at 525 nm is measured, and the amount of hydroxamic acid is calculated from a calibration curve. The amount of oxygen with which 1 M of hydroxamic acid is formed for 1 minute is defined as 1 unit (1U), which is the unit of transglutaminase activity.

The high molecular weight substance containing the amino acid donor and having the ability to bind the nucleic acid in accordance with the present invention may be any substance containing an amino group in a molecule and having the ability to bind a nucleic acid, especially a DNA molecule. It is preferable that the substance satisfy the following conditions:
i) a strong ability to protect DNA from decomposition;
ii) not caught non-specifically at any portion of a circulatory system in the human body;
iii) harmless to the human body; and
iv) does not act harmfully when the DNA enters the cell in combination with the receptor and is expressed within the cell.

The substance that satisfies such conditions is preferably a peptide or protein containing an amino group. Since a DNA plasmid is generally negatively-charged, a positively-charged high molecular weight substance can be mentioned. Its molecular weight is preferably in the range of from 5,000 to 200,000, more preferably from 10,000 to 100,000. Most preferable is polylysine.

Any biologically-active peptide which contains glutamine in the amino acid sequence and which is specifically bound to tissues, organs, cells or the like can be used in the present invention. The above-mentioned biologically-active peptides or proteins are preferable. Interleukin-2 and interleukin-6 are more preferable.

Possible uses of the present invention are mentioned below. In this regard, the particular biologically-active peptide is used as a targeting molecule. Thus the conjugate can be targeted to a particular target cell/organ and have a therapeutic purpose at that site.

### 1) Targeting molecule:

IL-2 or anti-IL-2 receptor antibody (antibody against α-chain, β-chain or γ-chain).

### Target cell, organ and therapeutic purpose:

a) An IL-2 receptor is sometimes expressed in a specific leukaemia cell, such as ATL. This cell can be eliminated.
b) When an organ or a myeloid cell is transplanted, rejection occurs. An activated T-cell causes this rejection, and this T-cell expresses an IL-2 receptor. This cell can be eliminated.
c) In chronic rheumatism, a lymphocyte that expresses an IL-2 receptor is locally present and precipitates the production of an auto-antibody, which leads to an attack or the progression of the disease. This lymphocyte can be eliminated.

### 2) Targeting molecule:

IL-6 or anti-IL-6 receptor antibody (antibody against gp80 or gp130)

Target cell, organ and therapeutic purpose:
a) Leukaemia cell having an IL-6 receptor. This leukaemia cell can be eliminated.
b) In chronic rheumatism, a lymphocyte that expresses an IL-6 receptor is locally present and precipitates production of an auto-antibody, which leads to an attack or the progression of the disease. This lymphocyte can be eliminated.

### 3) Targeting molecule:

Anti-megakaryocyte antibody

### Target cell, organ and therapeutic purpose:

Cells having a megakaryocyte-specific antigen. With respect to a thrombocytopenia, a gene such as the gene for IL-6, IL-11, mpl-ligand or the like can be transferred into a megakaryocyte to induce selective proliferation and maturation of the megakaryocyte.

### 4) Targeting molecule:

Anti-CD34 antibody

### Target cell, organ and therapeutic purpose:

Myeloid cells having CD34. A genetically-deficient gene, for example, ADA (Adenosine deaminase) can be transferred into the myeloid cell to treat the genetic disease. Further, a multi-drug-resistant (MRD) gene can be transferred into a bone marrow cell to make it drug-resistant, after which the cancer can be treated by chemotherapy.

### 5) Targeting molecule:

Anti-CD4 antibody

### Target cell, organ and therapeutic purpose:

Cells having CD4. A gene that suppresses the proliferation of HIV can be transferred into a HIV-infected cell to treat AIDS.

### 6) Targeting molecule:

Polymeric HSA or anti-albumin receptor antibody

### Target cell, organ and therapeutic purpose:

Polymeric HSA binds to a liver albumin receptor. A hepatoma can be treated by transferring an anti-oncogene corresponding to the hepatoma.

### 7) Targeting molecule:

Anti-ErbB2 antibody

### Target cell, organ and therapeutic purpose:

Cancer having ErbB2. The cancer can be treated by transferring an anti-oncogene into cancer cells.

### 8) Targeting molecule:

EGF

### Target cell, organ and therapeutic purpose:

An anti-oncogene can be transferred into cancer cells having an EGF-receptor of an ovarian cancer or the like to treat the cancer.

### 9) Targeting molecule:

Antibody against an antigen specific for a cancer of the large intestine, colon and rectum.

### Target cell, organ and therapeutic purpose:

An anti-oncogene can be transferred into a cell of a cancer of the large intestine, colon and rectum to treat the cancer.

In one embodiment of the present invention, the positively-charged high molecular weight substance binds the negatively-charged DNA molecule in an aqueous solution to protect the DNA molecule. It is possible that the conjugate obtained by linking the high molecular weight substance to the targeting molecule (or biologically active peptide) is mixed with the DNA molecule, such as a gene, to form a complex in which the DNA binds to the targeting molecule. By in vivo administration, this complex can specifically transfer the gene into a tissue corresponding to the target molecule, thus making it possible to produce the protein of that gene in that tissue. In the following Examples, polylysine is used as the high molecular weight substance. However, the molecule that is capable of binding DNA is not limited to polylysine. Any substance which has some affinity for DNA, which is not harmful to the human body and which is unlikely to be trapped in some portion of a circulatory system can be used.

Examples of the conjugate of the biologically-active peptide or protein and the substance having the ability to bind the nucleic acid, and examples of the complex of the conjugate and the nucleic acid in accordance with the present invention are shown below, but the conjugate and the complex of the present invention are not limited thereto.
1) Polylysine-IL-6/thymidine kinase gene expression plasmid: a) treatment of chronic rheumatism by transferring a suicide gene into an IL-6 receptor expression synovial membrane cell; and b) treatment of a cancer having an IL-6 receptor, for example, myeloma by inserting the suicide gene therein.
2) Polylysine-anti-CD14 antibody/ADA gene expression plasmid: Treatment of an SCID patient by transferring an ADA gene into a haematopoietic stem cell.
3) Polylysine-anti-CD14 antibody/β-globulin gene expression plasmid: Treatment of thalassaemia by introducing a normal β-globulin gene into a haematopoietic stem cell.
4) Polylysine-anti-CD4 antibody/thymidine kinase expression plasmid: Treatment of AIDS by transferring a suicide gene into an HIV-infected T-cell.
5) Polylysine-anti-CD4 antibody/reverse transcriptase anti-sense gene expression plasmid: Treatment of AIDS by transferring an HIV proliferation suppressive gene into a HIV-infected T-cell.

Using a transglutaminase, a biologically-active peptide or protein containing glutamine can be linked to a high molecular weight compound containing an amino group and having the ability to bind a nucleic acid (DNA or the like), via a glutamine residue of the peptide or protein under specific, mild conditions. Since this conjugate can bind the nucleic acid without losing any of the properties of the biologically-active peptide or protein, the nucleic acid can be transferred into cells selectively at high efficiency, utilizing the properties of the biologically-active peptide or protein which is specifically accumulated in tissues, organs, cells or the like. Accordingly, the present invention can be applied to gene therapy and the like.

The present invention will be described in more detail with reference to the following non-limiting Examples.

### Example 1 - Preparation of polylysine-bound IL-6 using polylysines having different molecular weights

Polylysine bromides having different molecular weights (7900, 45700 and 83800, made by Sigma Co.) were used in this Example.

10 mg of the respective polylysine bromides was each dissolved in 4 ml of 50 mM tris-HCl (pH 7.5). To the solution was added 50 µl of a BTG solution (14 U/ml of 50 mM tris-HCl, pH 7.5) . The resulting solution was allowed to stand at room temperature for 30 minutes and then 2 ml of an rhIL-6 (recombinant human IL-6) solution (2 mg/ml of 10 mM sodium citrate, pH 7.0) was added. The mixture was stirred, and then allowed to stand at 37 °C for 2.5 hours in the case of polylysine having a molecular weight of 7900, or for 20 hours in the case of polylysine having a molecular weight of 45700 or 83800.

The reaction solution was purified by reverse-phase HPLC (column = Vydac Protein C4 214TP54 (4.6 x 250 mm); solvents = A: 0.1% trifluoroacetic acid (TFA), and B: 0.1% TFA-80% acetonitrile; flow rate = 1 ml/min). The elution program of the chromatography is shown in Table 1.

**Table 1 -**

| Elution program | | |
|---|---|---|
| Time (min) | A % | B % |
| 0.0 | 100 | 0 |
| 5.0 | 100 | 0 |
| 5.1 | 60 | 40 |
| 35.0 | 0 | 100 |
| 50.0 | 0 | 100 |

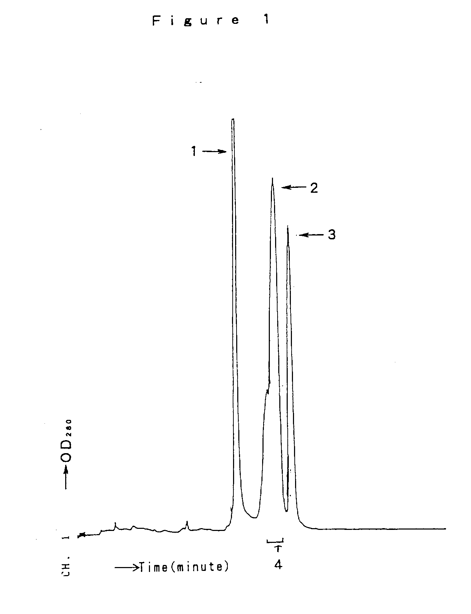

The chromatographic trace of the reaction solution when using polylysine having a molecular weight of 7900 is shown in Fig. 1, where peak 1 (22.14 minutes) indicates polylysine, peak 2 (27.57 minutes) indicates polylysine-bound rhIL-6, and peak 3 (29.66 minutes) indicates rhIL-6. Reference numeral 4 indicates a polylysine-bound rhIL-6 fraction.

The polylysine-bound IL-6 fraction was collected, and concentrated through the same reverse-phase HPLC. Elution was conducted upon linearly increasing the concentration of B buffer in A buffer from 0% to 100% over 5 minutes (A and B solvents (buffers) were the same as those used in the HPLC described above) . The polylysine-bound IL-6 fraction was further purified using gel filtration chromatography (Column = Sephadex G-75 (1 x 10 cm); Buffer = 20 mM tris-HCl, 0.5M NaCl (pH 8.5); Flow rate = 2 ml/min).

The purity of the thus-purified polylysine-bound IL-6 was measured by HPLC (Column = Vydac Protein C4 214TP54 (4.6 x 250 mm); Solvents = A: 0.1% trifluoroacetic acid (TFA), B: 0.1% TFA-80% acetonitrile; Flow rate = 1 ml/min.). The elution program is shown in Table 2.

**Table 2 -**

| Elution program | | |
|---|---|---|
| Time (min) | A % | B % |
| 0.0 | 60 | 40 |
| 20.0 | 0 | 100 |

All of the purified polylysine-bound IL-6 showed approximately single peaks.

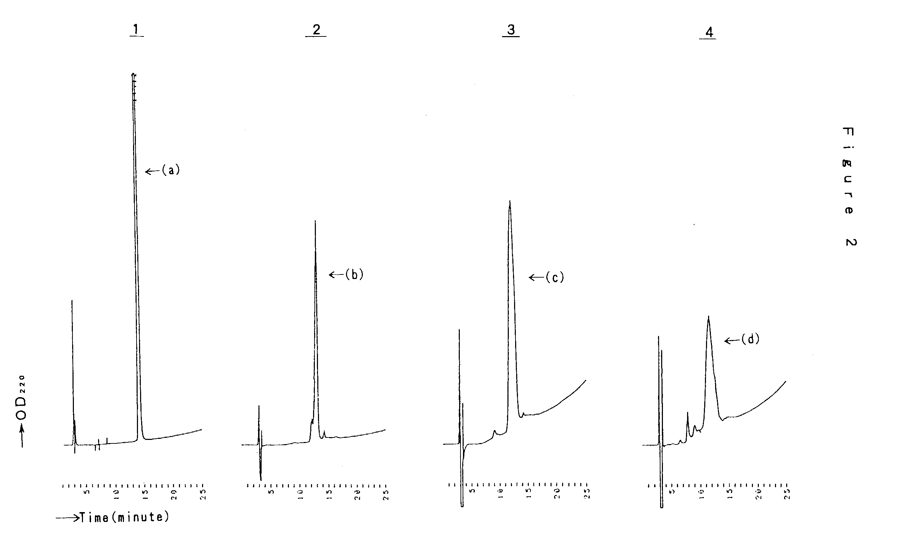

The pattern of this chromatography is shown in Fig. 2. In Fig. 2-1, peak (a) indicates rhIL-6 (retention time 14.12 minutes); in Fig. 2-2, peak (b) indicates rhIL-6 bound to polylysine having a molecular weight of 7900 (retention time 12.38 minutes); in Fig. 2-3, peak (c) indicates rhlL-6 bound to polylysine having a molecular weight of 45700 (retention time 12.16 minutes); in Fig. 2-4, peak (d) indicates rhIL-6 bound to polylysine having a molecular weight of 83800 (retention time 11.46 minutes).

Thus BTG was shown to have the ability to link all of the polylysines having the different molecular weights to IL-6.

### Example 2

### i) In-vitro activity of polylysine-bound IL-6

A MH60.32 strain was used which was obtained by sub-cloning IL-6-dependent mouse hybridoma MH60 BSF2 (Mazuda T. et al., Eur J. lmmunol, 18, 951, 1988).

The following solution was added to each well of a 96-well plate: a) 100 µl of a sample solution which was stepwise diluted in a culture medium; and b) 5 x 10³ MH60 hybridomas which were washed three times in a culture medium and suspended in 100 µl of the culture medium.

After the hybridomas were cultivated in the presence of 5% CO₂ at 37 °C for 60 hours, the number of cells was measured using an MTT assay, conducted as follows.

50 µl of an MTT solution (3- (4,5-dimethylthiazol-2-yl)-2,5-diphenyltetrazolium bromide in an amount of 1 mg/ml PBS solution) was added to each well. After completion of the cultivation, 150 ml of a supernatant was removed. To the residue was added 100 µl of an MTT solution (20% SDS 0.04N HCl solution), and the mixture was cultivated overnight. The absorbance (at from 570 nm to 630 nm) was measured by a microplate reader.

The culture media used in Example 2 were PRMI1640 (Gibco) and 10% FCS (inactive).

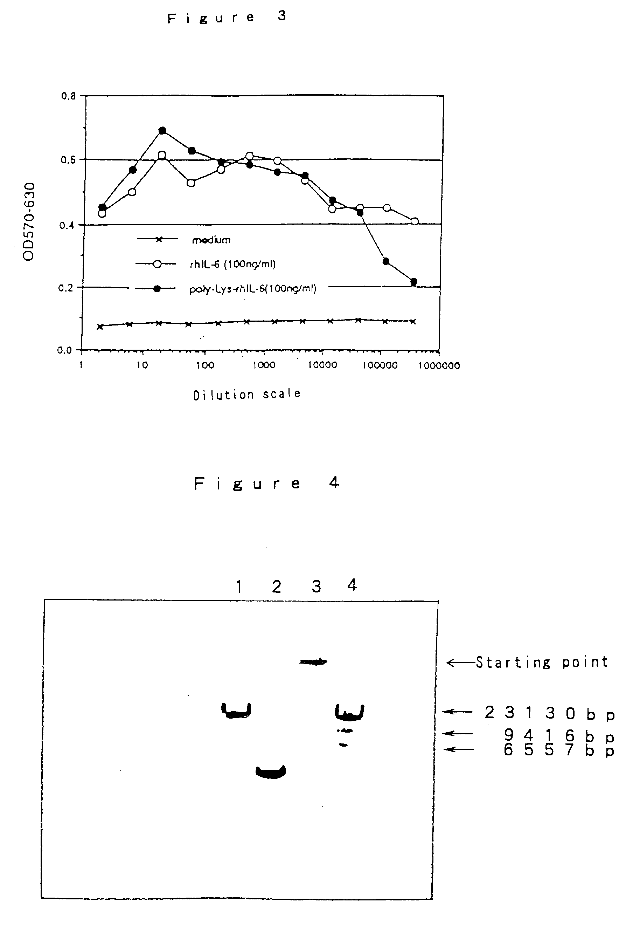

With respect to the proliferation activity of MH60, the results of the in-vitro activity of polylysine-bound IL-6 (molecular weight 7900) are shown in Fig. 3. From the results, it follows that the activity of polylysine-bound IL-6 was equal to that of IL-6. Polylysine-bound IL-6 which was bound to polylysine having a molecular weight of 45700 or 83800 also exhibited an activity which was equal to that of IL-6.

### b) In-vivo activity of polylysine-bound IL-6

A sample (0.1 ml) was subcutaneously injected into healthy ICR-strain mice which were approximately 6 weeks old and had a weight of 25 g. The sample was diluted with PBS and suspended in PBS. One group of test animals consisted of 5 animals. The sample was administered twice a day (day and night) and for a total of 9 times until the morning of Day 5. After 4 to 8 hours from the last administration, blood was collected from the heart of each test animal, and was mixed with an anticoagulant. Within 3 hours from the collection of blood, the number of platelets was counted using a method of detecting a change in resistivity. The average number of platelets per unit volume was counted.

The results in the case of using polylysine-bound IL-6 which was linked to polylysine having a molecular weight of 7900 are shown in Table 3.

**Table 3 -**

| In-vivo activity of polylysine rhIL-6 (increase in number of platelets) | |
|---|---|
| Medicament | Number of platelets ± S.D. ( x 10⁴/µl) |
| Citrate Buffer (10 mM sodium citrate, pH 7.0) | 111.2 ± 8.9 |
| rhIL-6 (5 µg/shot) | 124.6 ± 19.1 |
| Polylysine rhIL-6 (5 µg/shot) | 131.2 ± 7.7 |

As is clear from Table 3, polylysine-IL-6 increased the number of platelets in vivo compared to IL-6.

Polylysine-bound IL-6 which was bound to polylysine having a molecular weight of 45700 or 83800 exhibited the same activity as that shown in Table 3.

### Example 3 - Formation of a composite of polylysine-bound IL-6 and a DNA

100 µl of polylysine-bound IL-6 (0.3 mg/ml of 0.2M sodium phosphate, pH 6.0) was added to 1 µl of a DNA solution (2 µg/µl). The mixture was stirred by means of a vortex stirrer for 15 seconds, and then allowed to stand at room temperature for 30 minutes to form a composite or complex of the two components. The complex of polylysine-bound IL-6 and DNA, and DNA alone were electrophoresed on a 0.8% agar gel to confirm the formabation of the complex by ultraviolet light in the presence of EtBr.

The pattern of the electrophoresis is shown in Fig. 4, where 1 and 4 are markers (digested with X-Hind III), 2 is a plasmid vector (pSV-β-galactosidase vector), and 3 is a complex of DNA and polylysine-bound IL-6.

As can be seen in Fig. 4, when the DNA formed a complex with polylysine-bound IL-6, it was not electrophoresed on the 0.8% agar gel and stayed at the original point. This is because polylysine-bound IL-6 prepared by using BTG formed a complex with DNA. Thus, polylysine-bound IL-6 proved to have the ability to form a complex with the DNA.

In Fig. 4, polylysine-bound IL-6 which was linked to polylysine having a molecular weight of 7900 was used. When using polylysine-bound IL-6 which was bound to polylysine having a molecular weight of 45700 or 83800, the same results were also achieved.

### Example 4 - Specific transfer of a gene into an IL-6 receptor-containing cell by means of polylysine-bound IL-6 (1)

A complex comprising 20 µg of a DNA and 10 µg of polylysine-bound IL-6 which was prepared by the same method as in Example 3 was added to cultured cells. Sub-cultured cells were charged into 2 ml of a culture media on 1 x 10⁵ 6-well plates, and were cultured for some hours. The above-mentioned polylysine-bound IL-6/DNA complex was added thereto. The mixture was cultured in the presence of 5% CO₂ at 37 °C for 48 hours. Then, the expression of β-galactosidase was measured according to a Technical Bulletin of Promega (reference literature, F.C. Lucibello et al., Met. Mol. Cell Biol., vol. 1, 9, 1989).

With respect to the cells, U266 was used as an IL-6 receptor-containing cell, and CEM as a cell free from IL-6 receptors, respectively. pSV-β-galactosidase vector (made by Promega, USA) was used as the DNA.

The results are shown in Table 4. As is apparent, the IL-6 receptor-containing cell U266 exhibited significantly higher β-galactosidase activity compared to the IL-6 receptor-free cell CEM.

Further, in the U266 cell, the β-galactosidase activity of the cell cultured with the complex of polylysine-bound IL-6 and DNA was higher than that of the cell cultured with polylysine-bound IL-6 or DNA alone.

**Table 4 -**

| β-galactosidase (lacZ) gene transfer by means of polylysine IL-6 | | |
|---|---|---|
| Culture medium for growth of cells | Absorbance at 405-595 nm | |
| | U266 | CEM |
| Blank | 0.08 | 0.07 |
| Culture Medium as such | 0.30 | 0.25 |
| DNA-containing culture medium | 0.29 | 0.23 |
| Culture containing polylysine (molecular weight 7900)-bound rhIL-6 | 0.34 | 0.24 |
| Culture containing polylysine (molecular weight 45700)-bound rhIL-6 | 0.25 | 0.23 |
| Culture containing polylysine (molecular weight 83800)-bound rhIL-6 | 0.35 | 0.25 |
| Culture containing polylysine (molecular weight 7900)-bound rhIL-6/DNA complex | 1.08 | 0.24 |
| Culture containing polylysine (molecular weight 45700)-bound rhIL-6/DNA complex | 1.10 | 0.25 |
| Culture containing polylysine (molecular weight 83800)-bound rhIL-6/DNA complex | 1.03 | 0.23 |

### Example 5 - Specific transfer of a gene into an IL-6 receptor-containing cell by means of polylysine-bound IL-6 (2)

Polylysine-bound IL-6 and a complex of a DNA and polylysine-bound IL-6 were prepared in the same manner as in Example 3. Cells were cultured in a DNA-containing culture medium, a culture medium containing polylysine-bound IL-6 and a culture medium containing a complex of the DNA and polylysine-bound IL-6. The amounts added of the DNA, polylysine-bound IL-6 and the composite of the DNA and polylysine-bound IL-6, and the cultivation method were the same as in Example 3. U266 or U937 was used as the IL-6 receptor-containing cell. Hepg2 or Hep3B was used as the IL-6 receptor-free cell.

The molecular weight of polylysine in the polylysine-bound IL-6 used was 7900. The DNA used was pSV2CAT (Gorman, C.M. et al., Mol. Cell Biol., 2, 1044, 1982).

Chloramphenicol acetyltransferase (CAT) activity was measured by the method of Murray (Murray, I.A., et al., Nucleic Acids Res., 19, 6648, 1991).

The results are shown in Table 5, from which it is apparent that the cells which were cultured by adding the complex of the DNA and polylysine-bound IL-6 exhibited significantly higher CAT activity compared to that of the cells which were cultured by adding DNA or polylysine-bound IL-6 alone.

**Table 5 -**

| CAT gene transfer by means of polylysine IL-6 | | | | |
|---|---|---|---|---|
| Culture medium for growth of cells | CPM x10³ | | | |
| | U266 | U937 | HepG2 | Hep3B |
| Blank | 0.1 | 0.1 | 0.1 | 0.1 |
| Culture Medium as such | 3.0 | 2.2 | 4.5 | 3.5 |
| DNA-containing culture medium | 4.3 | 2.3 | 5.0 | 4.5 |
| Culture containing polylysine-bound rhIL-6 | 3.2 | 3.3 | 4.5 | 3.5 |
| Culture containing polylysine-bound rhIL-6/DNA complex | 25.2 | 15.2 | 10.0 | 12.0 |

## Claims

1. A conjugate of (a) a biologically-active peptide or protein having at least one glutamine residue and (b) a high molecular weight substance containing an amino acid donor and having the ability to bind a nucleic acid, the biologically-active peptide or protein being linked to the high molecular weight substance by an acid-amide linkage between the amino group of the amino acid donor and the γ-position of the glutamine residue.

2. A conjugate as claimed in claim 1, wherein the high molecular weight substance is polylysine.

3. A conjugate as claimed in claim 1 or claim 2, wherein the biologically-active protein is interleukin-6.

4. A conjugate as claimed in claim 1 or claim 2, wherein the biologically-active protein is interleukin-2.

5. A process for producing a conjugate as defined in any preceding claim, comprising reacting said biologically-active peptide or protein with said high molecular weight substance in the presence of a transglutaminase.

6. A process as claimed in claim 5, wherein the transglutaminase is derived from a microorganism.

7. A complex comprising the conjugate as claimed in any one of claims 1 to 4 or produced by the method of claim 5 or claim 6, and a nucleic acid.

8. A complex as claimed in claim 7, wherein the nucleic acid is DNA.

9. A pharmaceutical composition for gene transfer therapy, which comprises the conjugate as claimed in any one of claims 1 to 4 or produced by the method of claim 5 or claim 6, and a pharmaceutically-acceptable carrier.

10. A pharmaceutical composition for gene transfer therapy, which comprises the complex as claimed in claim 7 or claim 8, and a pharmaceutically acceptable carrier.
